# EUROPEAN PATENT SPECIFICATION

(11) **EP 3 463 774 B1**
(45) Date of publication and mention of the grant of the patent: **05.10.2022**
(21) Application number: 17726466.0
(22) Date of filing: 19.05.2017
(51) Int. Cl.: B26D 7/20, B29C 65/00, B26F 1/38, B29C 65/08, B26D 7/01, A61F 13/15, B23K 26/324, B23K 26/34, B23K 26/342, B23K 103/00

(54) **ROTARY ANVIL**
DREHAMBOSS
ENCLUME ROTATIVE

(30) Priority: 24.05.2016 US 201662340569 P
(43) Date of publication of application: 10.04.2019
(73) Proprietor: The Procter & Gamble Company, Cincinnati, OH 45202 (US)
(72) Inventor: SCHNEIDER, Uwe, Cincinnati, Ohio 45202 (US); EIMANN, Klaus, 97828 Markheidenfeld (DE); VANVALKENBURG, Curtis, Hunter, Cincinnati, Ohio 45202 (US)
(74) Representative: P&G Patent Germany
(86) International application number: PCT/US2017/033460
(87) International publication number: WO 2017/205195

(56) References cited:
- EP-A1- 2 042 437
- EP-A2- 2 279 954
- WO-A1-97/23398
- CN-U- 204 658 567
- DE-A1- 10 209 214
- DE-A1- 19 748 110
- DE-A1-102006 017 086
- US-A- 5 259 283
- US-A- 5 983 764
- US-A1- 2012 079 926
- US-A1- 2013 049 438
- US-A1- 2014 377 506
- Steffen Nowotny, Lutz-Michael Berger, Jörg Spatzier: "1.18 Coatings by Laser Cladding" In: Vinod. K Sarin, Editor-in-Chief: "Comprehensive Hard Materials", 19 March 2014 (2014-03-19), Elsevier, Amsterdam, Waltham, heidelberg, London, New York, Oxford, Paris, San Diego, San Francisco, Singapore, Sidney, Tokyo, XP00919942, ISBN: 978-0-08-096528-4 pages 507-525, DOI: https://doi.org/10.1016/B978-0-08-096527-7 .01001-1, page 507 - page 525

## Description

### FIELD OF THE INVENTION

The present disclosure relates to rotary anvils having a body constructed from a first material with abrasion resistant material fused to the body, wherein the abrasion resistant material is different from the first material.

### BACKGROUND OF THE INVENTION

Along an assembly line, various types of articles, such as for example, diapers and other absorbent articles, may be assembled by adding components to and otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. Webs of material and component parts used to manufacture diapers may include: backsheets, topsheet, absorbent cores, front and/or back ears, fastener components, and various types of elastic webs and components such as leg elastics, barrier leg cuff elastics, and waist elastics. Once the desired component parts are assembled, the advancing web(s) and component parts are subjected to a final knife cut to separate the web(s) into discrete diapers or other absorbent articles. The discrete diapers or absorbent articles may also then be folded and packaged.

Various methods and apparatuses may be used for forming and/or attaching different components to an advancing web and/or otherwise modify an advancing web during the manufacturing process. For example, some operations may utilize a rotary knife and anvil roll to cut advancing webs into discrete components. As such, a continuous web may advance between a rotating knife and rotating anvil roll. As the knife rotates, a blade contacts the continuous web against the anvil roll and severs a discrete component from the continuous web. Repetitive contact between the blade and anvil roll causes wear on the anvil roll. In some configurations, the knife roll and the anvil roll may be sized such that the blade contacts the anvil roll in the same location during each cut. Thus, the anvil roll may exhibit localized wear in these locations, necessitating repair or replacement of the anvil roll.

In turn, various steps may be taken to help increase the life of the anvil roll and reduce the frequency at which repairs may be needed. For example, to help mitigate problems associated with excessive localized wear on the anvil roll, the knife roll and anvil roll may be sized and operate such that the knife blade contacts the anvil roll in different locations. Contacting the anvil roll in different locations may help to provide relatively more even wear on the outer circumferential surface of the anvil roll, which may increase the period of time between anvil roll repairs and replacement. However, with some types of anvil roll designs, it may be difficult to configure the blade to contact the anvil roll in different locations during operation. For example, the anvil roll may also be configured as a vacuum drum that applies vacuum pressure to maintain the position of a discrete component cut from the web on the outer surface of the anvil roll. As such, the outer surface of the anvil roll may be configured with vacuum holes connected with a vacuum source adapted to apply vacuum pressure to the discrete component. In turn, it may be necessary to have the blade contact the anvil roll between the vacuum holes. The repetitive contact of the blade against the anvil roll in locations between the vacuum holes may increase the likelihood of localized wear on anvil roll surface.

In some configurations, the anvil roll may be made from relatively hard materials, such as tungsten carbide, to help increase the life the anvil roll. However, such materials may be relatively costly, and the increased hardness of such materials may increase the difficulties associated with machining anvil rolls during manufacture. For example, it may be relatively difficult to machine vacuum holes in anvil roll made from such hard materials. In some instances, it may desirable to apply such hard materials only to localized areas of the anvil roll, such as where a blade contacts the anvil roll during operation. For example, the anvil roll may be designed such that pieces of hard materials are bolted to a base surface of the anvil roll. However, precisely machining such pieces of hard materials and base surfaces of the anvil roll may increase the expenses and complexities associated with manufacturing such anvil rolls.

Consequently, it would be beneficial to provide apparatuses with vacuum anvil rolls that are less susceptible to localized wear and wherein the anvil rolls may be designed for ease of manufacture at relatively low costs.

An apparatus as defined by the preamble of claim 1 is known for example from EP 2 279 954 A2.

### SUMMARY OF THE INVENTION

The present disclosure relates to an apparatus as defined in claim 1.

### BRIEF DESCRIPTION OF THE DRAWINGS

Figure 1 is an isometric view of an anvil roll.
Figure 1A is a side view of an outer circumferential surface of the anvil roll in Figure 1A.
Figure 1B is a side view of the anvil roll from Figure 1A taken along line 1B-1B.
Figure 1C is a sectional view of the anvil roll from Figure 1A taken along line 1C-1C.
Figure 2 is an isometric view of a forging of a body.
Figure 3 is an isometric view of a body.
Figure 3A is a side view of an outer circumferential surface of the body in Figure 3A.
Figure 3B is a side view of the body from Figure 3A taken along line 3B-3B.
Figure 3C is a sectional view of the body from Figure 3A taken along line 3C-3C.
Figure 4A is a side view of an outer circumferential surface of the body with a second strip configuration.
Figure 4B is a side view of an outer circumferential surface of the body with a third strip configuration.
Figure 4C is a side view of an outer circumferential surface of the body with a fourth strip configuration.
Figure 4D is a side view of an outer circumferential surface of the body with a fifth strip configuration.
Figure 4E is a side view of an outer circumferential surface of the body with a sixth strip configuration.
Figure 4F is a side view of an outer circumferential surface of the body with a seventh strip configuration.
Figure 4G is a side view of an outer circumferential surface of the body with a second vacuum hole configuration.
Figure 4H is a sectional view of the body from Figure 4G taken along line 4H-4H.
Figure 5 is a schematic side view of a substrate advancing between an anvil roll and a tool member.
Figure 6 is a side view of the anvil roll and tool member from Figure 5 taken along line 6-6.^{∗}
Figure 7 is a detailed view of an anvil roll and a tool member configured as a cutting roll with an implement in the form of a blade.
Figure 8 is side view of an anvil roll with a body configured as an elongate member.
Figure 9 is side view of an anvil roll with a body configured as an elongate member.
Figure 10A is a side view of a body of an anvil roll configured vacuum channels.
Figure 10B is a side view of the body in Figure 10A with strips of abrasion resistant material fused thereto.
Figure 10C is a side view of the body and strips in Figure 10B with outer shells connected thereto.
Figure 11A is a partially cut away plan view of an absorbent article in the form of a taped diaper that may include one or more substrates and components manipulated during manufacture according to the apparatuses and methods disclosed herein with the portion of the diaper that faces away from a wearer oriented towards the viewer.
Figure 11B is a plan view of the absorbent article of Figure 11A that may include one or more substrates and components manipulated during manufacture according to the apparatuses and methods disclosed herein with the portion of the diaper that faces toward a wearer oriented towards the viewer.

### DETAILED DESCRIPTION OF THE INVENTION

The following term explanations may be useful in understanding the present disclosure:
"Absorbent article" is used herein to refer to consumer products whose primary function is to absorb and retain soils and wastes. "Diaper" is used herein to refer to an absorbent article generally worn by infants and incontinent persons about the lower torso. The term "disposable" is used herein to describe absorbent articles which generally are not intended to be laundered or otherwise restored or reused as an absorbent article (e.g., they are intended to be discarded after a single use and may also be configured to be recycled, composted or otherwise disposed of in an environmentally compatible manner).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

The term "substrate" is used herein to describe a material which is primarily two-dimensional (i.e. in an XY plane) and whose thickness (in a Z direction) is relatively small (i.e. 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

The present disclosure relates to apparatuses and methods for manufacturing absorbent articles, and more particularly, rotary anvils that may be used in combination with a tool member to perform various types of manufacturing operations, such as cutting, bonding, and embossing. Particular aspects of the present disclosure involve an anvil roll having a cylindrically-shaped outer circumferential surface and being adapted to rotate about a first axis of rotation. The anvil roll includes a body formed from a first material, such as a metallic material. Holes in the body may extend radially inward from the outer circumferential surface, wherein the holes are in fluid communication with a vacuum pressure source. Although anvils described herein may include vacuum, it is appreciated that anvils herein may be configured without vacuum. The body may also include one or more grooves in the outer circumferential surface. The grooves may extend in various directions and may also extend between at least two of the holes. In turn, one or more abrasion resistant materials may be fused to the body and fill the grooves to form one or more strips. The abrasion resistant material is different from the first material of the body. As discussed in more detail below, a tool member may be positioned adjacent the anvil roll and adapted to rotate about a second axis of rotation. During operation, the anvil roll and the tool member rotate in opposite directions such that the tool member contacts a portion of the outer circumferential surface of the anvil roll defined by the one or more strips of abrasion resistant material. As discussed in more detail below, because the strips of abrasion resistant material are formed on and fused to the body as opposed to being separately fabricated and/or fastened thereto, some of the difficulties associated with current anvil roll manufacturing techniques may be alleviated.

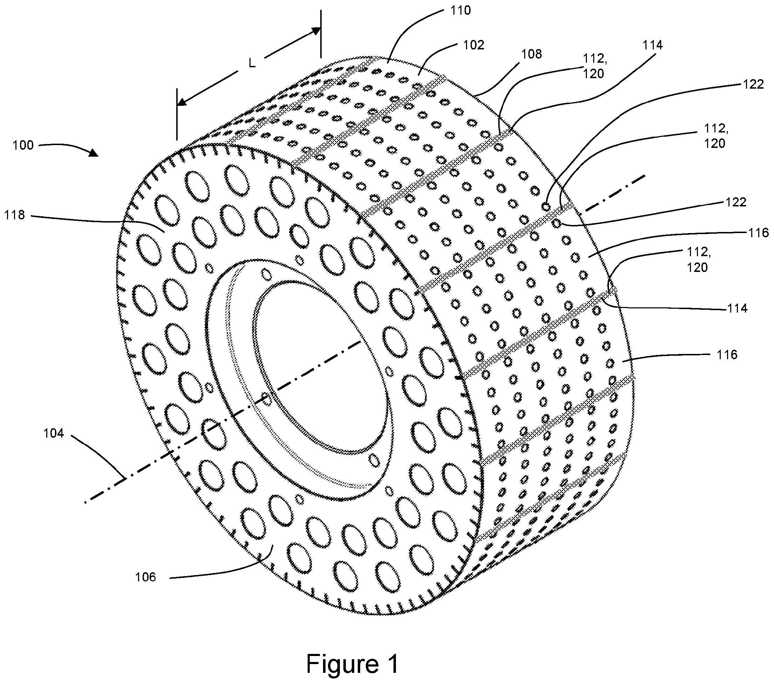

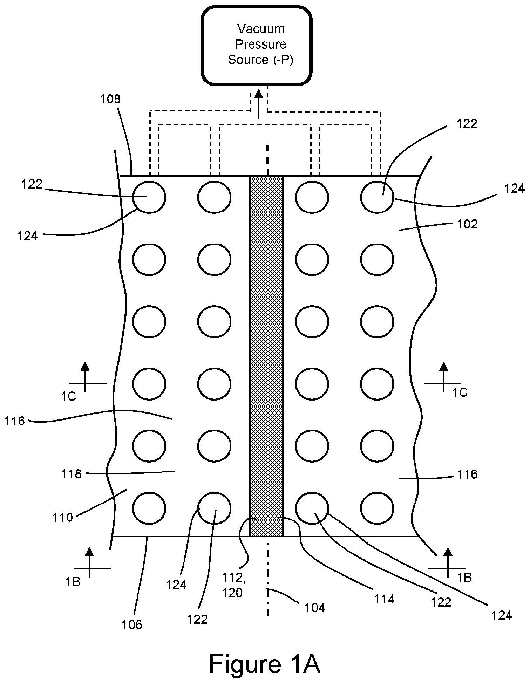

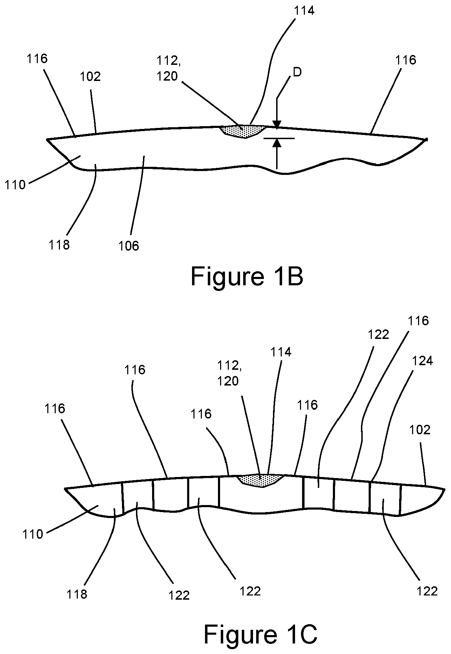

It is to be appreciated the anvil rolls herein may be configured in various ways. For example, Figure 1 shows an isometric view of a configuration of an anvil roll 100 having a cylindrically-shaped outer circumferential surface 102 and adapted to rotate about a first axis of rotation 104. The anvil roll 100 may extend axially for a length L between a first end 106 and a second end 108. As shown in Figures 1 and 1A, the anvil roll 100 also includes a body 110 and one or more strips 112 fused thereto, such that the one or more strips 112 define a first portion 114 of the outer circumferential surface 102 and the body 110 defines a second portion 116 of the outer circumferential surface 102. As discussed in more detail below, the body 110 may be configured as a unitary member made from a first material 118 and the strips 112 are made from one or more abrasion resistant materials 120 that are different from the first material 118. With reference to Figures 1, 1A, and 1C, the anvil roll 100 includes one or more holes 122 in the outer circumferential surface 102. More particularly, each hole 122 defines a perimeter 124 in the second portion 116 of the outer circumferential surface 102 and extends radially inward from the outer circumferential surface 102 into the body 110. As schematically represented in Figure 1A, a vacuum source 126 may be in fluid communication with the holes 122. As such, the vacuum source 126 may create vacuum air pressure in the holes 122 during operation to help hold substrates in a desired position on the outer circumferential surface 102 of the anvil roll 100.

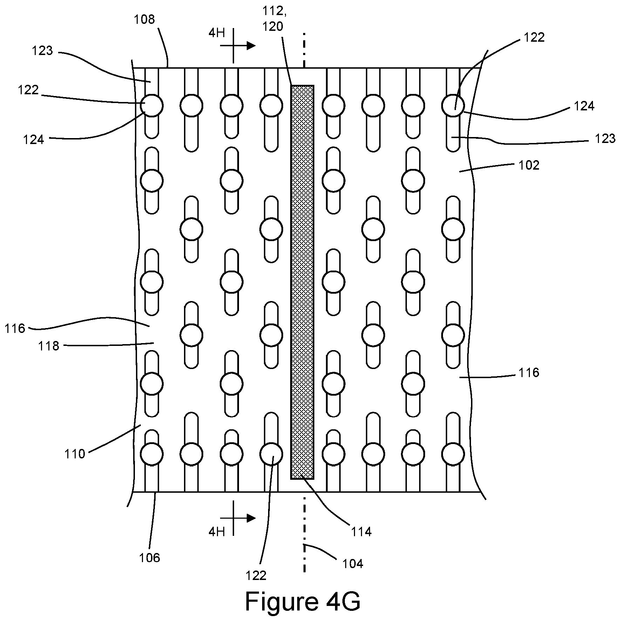

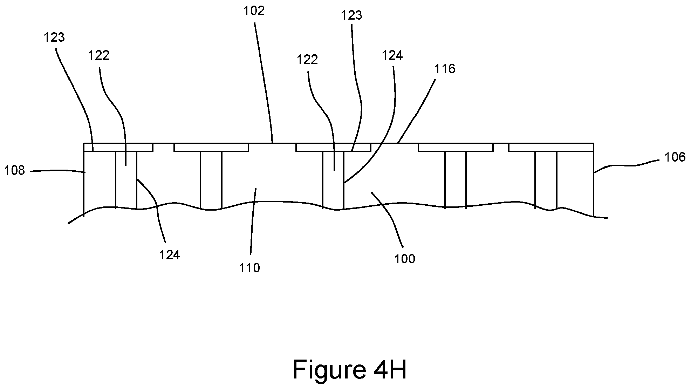

Although the holes 122 are sometimes depicted as being arranged in rows extending axially along the outer circumferential surface 102 of the anvil roll, it is to be appreciated that the holes 122 may be arranged in various ways and may be configured to have the same or different shapes and/or sizes, such as shown in Figures 4G and 4H for example. The holes 122 shown in Figures 4G and 4H may also include a recessed zone 123 extending radially inward from the outer circumferential surface 102. The recessed zone 123 may partially or completely surround the perimeter 124 of the hole 122. It is also to be appreciated that various types of one or more vacuum sources and arrangements thereof may be used with the anvil roll 100.

As previously mentioned, the first material 118 of the body 110 is different from the one or more abrasion resistant materials 120 fused thereto. It is to be appreciated that the first material 118 may be various types of materials, such as various types of metallic materials. For example, in some configurations, the first material 118 is selected from the group consisting of: an iron alloy, an aluminum alloy, and a titanium alloy. In some configurations, the iron alloy is selected from the group consisting of: stainless steel and tool steel. In some configurations, the first material is a hot-working tool steel or a tool steel, such as for example, X37CrMoVS-1 steel. It is also to be appreciated that the one or more abrasion resistant materials 120 may be various types of materials. For example, the one or more abrasion resistant materials 120 may include at least one of: powder-metallurgical steel; titanium carbide, niobium carbide, tantalum carbide, chromium carbide, tungsten carbide, and and mixtures thereof. The abrasion resistant material 120 may include a carbide of at least one element of the fourth, the fifth, the sixth and/or the seventh group of the periodic table. Carbides from the fourth group may be titanium carbide, zirconium carbide, hafnium carbide or a mixture thereof. Carbides from the fifth group may be vanadium carbide, niobium carbide, tantalum carbide or a mixture thereof. Carbides from the sixth group may be chromium carbide, molybdenum carbide, tungsten carbide or a mixture thereof. Carbides from the seventh group may be manganese carbide, rhenium carbide or a mixture thereof. Carbides of several groups can be used individually or as a mixture. In one embodiment, titanium carbide, niobium carbide, tantalum carbide, chromium carbide, tungsten carbide or a mixture thereof is used. The carbides may be deposited as a powder comprising particles of several sizes and/or shapes. Carbides may be provided in a matrix material, wherein matrix material may comprise nickel, cobalt and/or iron. The carbides may be present in the matrix material in an amount of from about 60% to about 80%, in another embodiment in an amount of from about 70% to about 80%, in yet another embodiment in an amount of from about 70% to about 75% or in yet another embodiment any individual number within the values provided or in any range including or within the values provided.

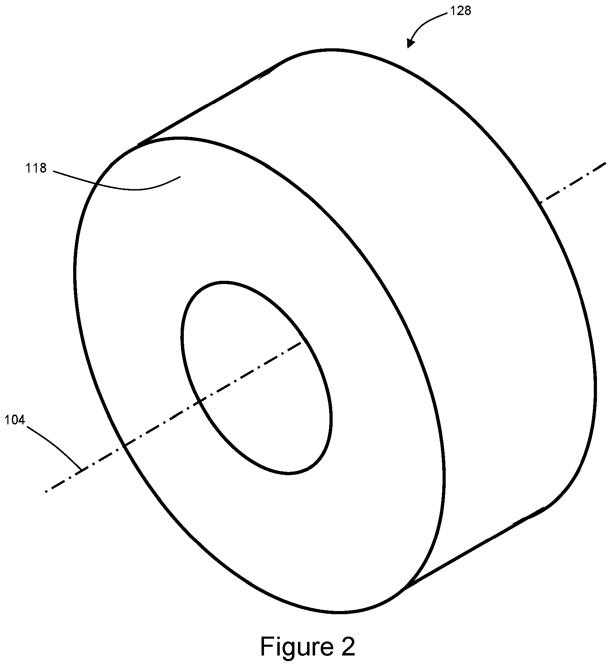

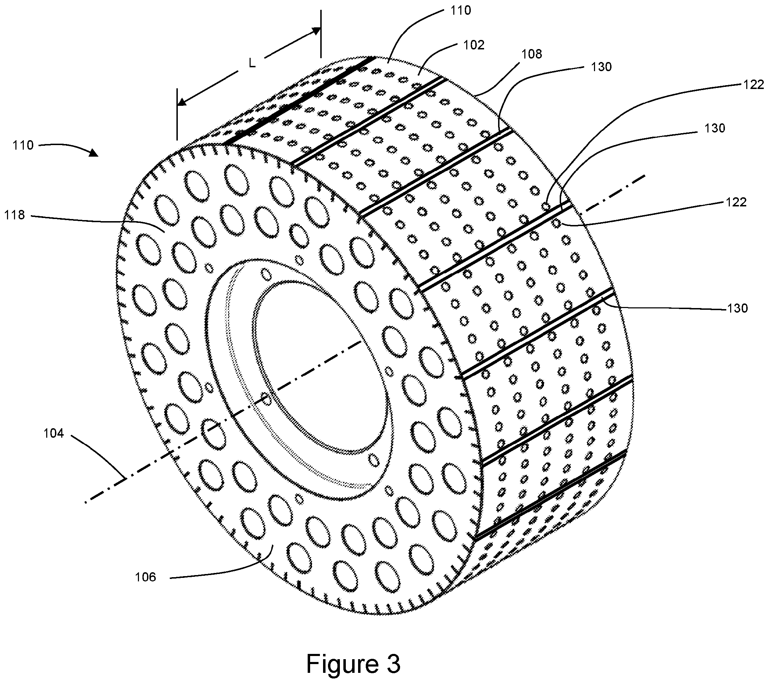

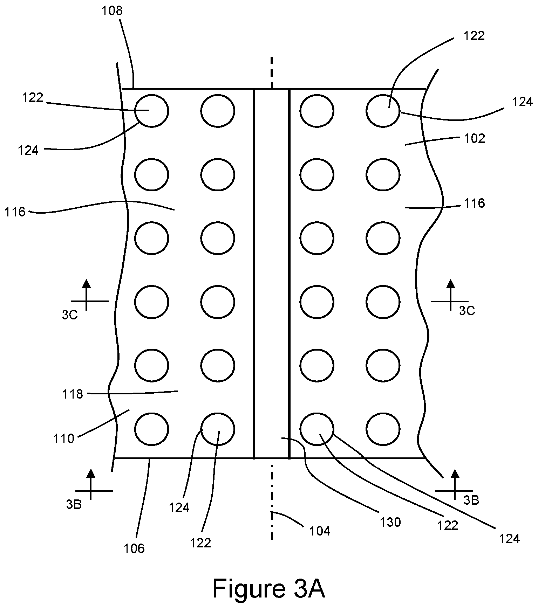

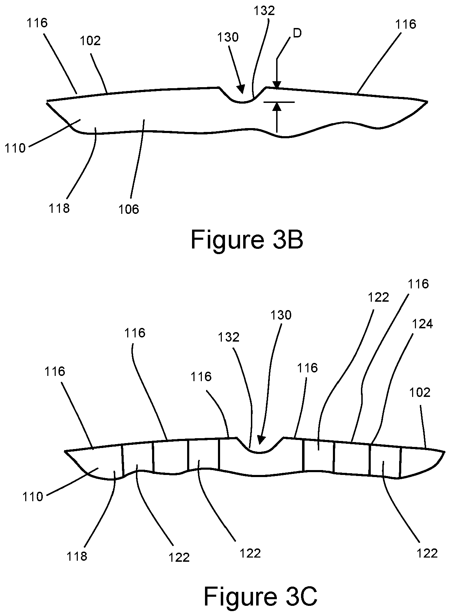

When assembling the anvil rolls 100 herein, the first material 118 may be formed into a generally cylindrically-shaped forging 128, such as shown in Figure 2. The forging 128 may be machined or otherwise worked to form the body 110. For example, the forging 128 may be worked or machined into the body 110 so as to include various features, such as holes 122 and grooves 130, such as shown Figures 3-3C.

Each groove 130 may be defined by a base surface 132 extending radially inward from the outer circumferential surface 102 of the body 110. It is to be appreciated that grooves 130 may have various shapes and sizes. For example, the grooves 130 may extend between and separate the perimeters 124 of two or more holes 122. In some configurations, one or grooves 130 may extend axially for less than or for the entire length L between a first end 106 and a second end 108 of the body 110. The grooves 130 may also define various radial depths D. In some configurations, the radial depth D of one or more grooves may be greater than about 2 mm and less than about 4 mm. In addition, the grooves may have the same or different shapes, sizes, and or radial depths. As discussed in more detail below, the abrasion resistant material 120 is deposited into the grooves 130 to form the strips 112 on the anvil roll 100 as shown for example in Figures 1-1C. More particularly, the abrasion resistant material 120 is fused to first material 118 of the body 110 without having to otherwise fasten such strips to the body. As such, the anvil rolls 100 herein may not require extensive machining of abrasion resistant materials and/or require assembly operations of fastening discrete components made of abrasion resistant materials to the body. Thus, some of the difficulties associated with assembling current anvil rolls may be alleviated.

As previously mentioned, one or more abrasion resistant materials 120 are fused to the body 110 in the grooves 130. And in some configurations, one or more abrasion resistant material 120 may be fused to the body 110 with a laser cladding process, such as disclosed in U.S. Patent Publication No. 2013/0049438 A1. During the laser cladding process, the first material 118 of the body 110 may be partially melted during deposition of the abrasion resistant material 120 into the grooves 130. As such, a metallurgic bond may be created between the abrasion resistant material 120 and the first material 118 of the body 110. As used herein, a "metallurgical bond" means that the abrasion resistant material is fused to the first material of the body such that the microstructure of the first material may be intimately linked to the microstructure of the abrasion resistant material. In some configurations when applying more than one abrasion resistant material, metallurgic bonds may be also created between the different abrasion resistant materials.

In some configurations, the abrasion resistant material may include multiple layers of material that are applied to the body 110, such as disclosed in U.S. Patent Publication No. 2013/0049438 A1. For example, the abrasion resistant material may include a first layer, a second layer, and a third layer, wherein the first layer may be referred to as a bonding layer, the second layer may be referred to as a bearing layer, and the third layer may be referred to as a wear resistant layer. The bonding layer may be applied to the body 110; the bearing layer may be applied to the bonding layer; and the wear resistant layer may be applied to the bearing layer. Thus, the first layer or bonding layer may provide a metallurgical bond to the body 110 when applied by a welding or laser cladding process. As such, the bonding layer may be a metal alloy that is similar to the first material 120 of the body 110, which in turn, may form little or no brittle phase when mixed with the first material 120. The second layer or bearing layer may be configured to provide sufficient strength and stiffness when the wear resistant layer is loaded during operation. Thus, the second layer or bearing layers may be a metallic alloy that is similar to the bonding layer but contains elements to form solid solutions and/or medium hard phases. The third layer or wear resistant layer may be a compound of a matrix in which hard phases, such as for example carbides, borides and/or nitrides, are embedded. The matrix may be a metallic alloy which is similar to the bearing layer but also contains elements to form a solid solution and/or medium hard phases, and also be identical with the bearing layer. The hard phases may be homogeneously distributed inside the metallic matrix in various amounts. The hard phases may also be incorporated as solid particles during the coating process or may precipitate during the solidification process from the melt.

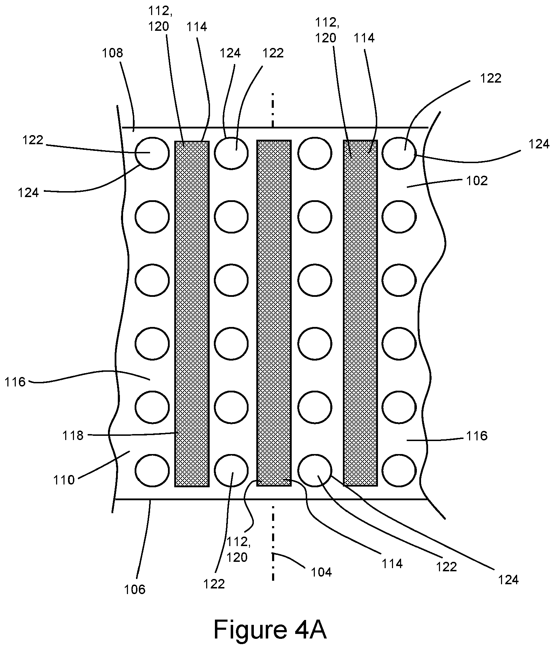

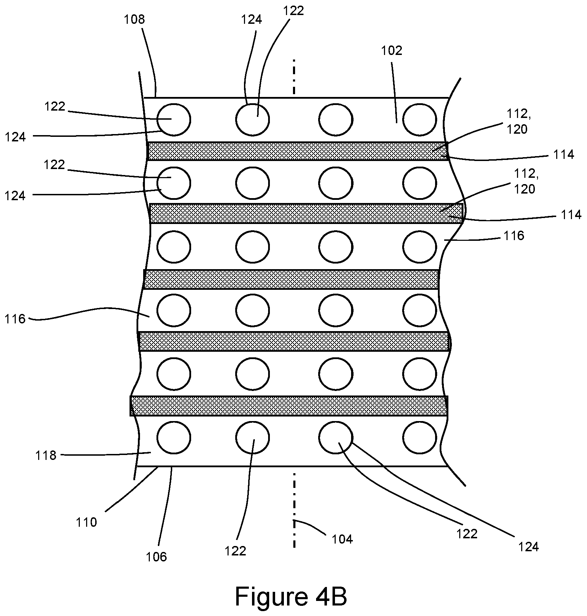

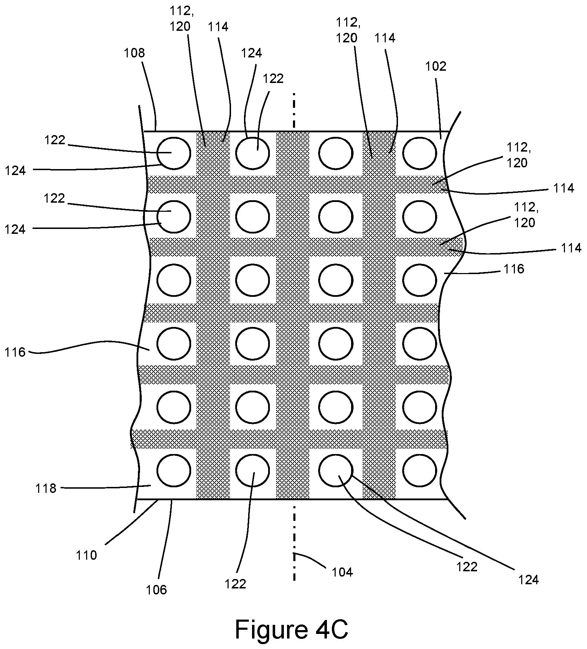

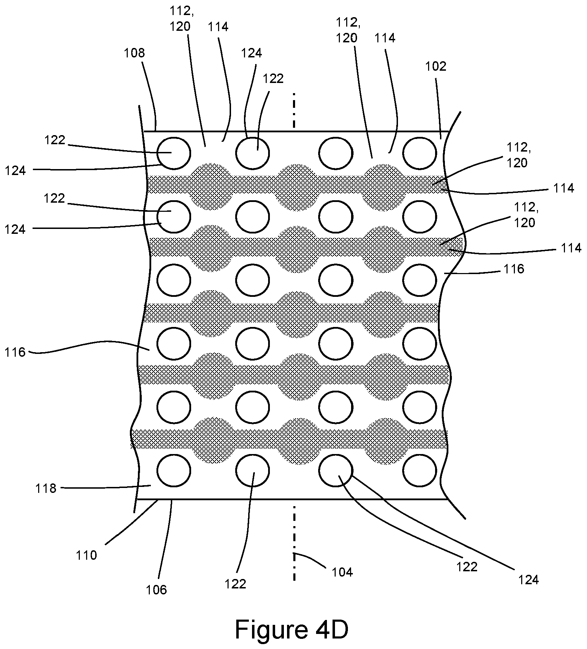

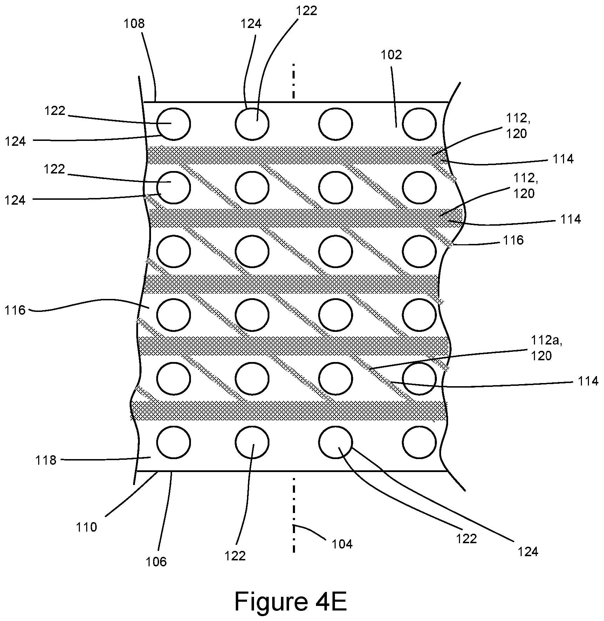

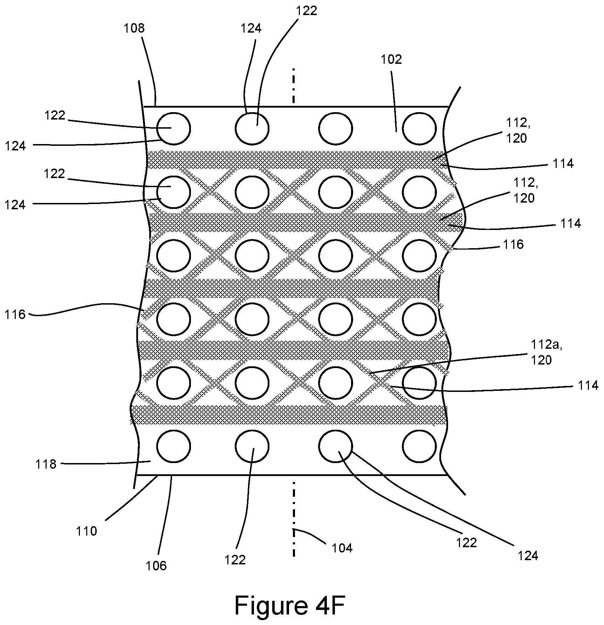

Because abrasion resistant materials 120 are deposited into and fill the grooves 130 to form the strips 112, it is to be appreciated that strips 112, as with the grooves 130, may have various shapes and sizes. For example, the strips 112 may have the same or different shapes, sizes, and/or radial depths. The strips 112 may define various radial depths D. In some configurations, the radial depth D of one or more strips 112 may be greater than about 2 mm and less than about 4 mm. For example, as shown in Figure 1A, the strips 112 may extend between and separate the perimeters 124 of two or more holes 122. Also as shown in Figure 1A, one or more strips 112 may extend axially for the entire length L between a first end 106 and a second end 108 of the body 110. In some configurations, one or more strips 112 may extend axially for less than the entire length L between a first end 106 and a second end 108 of the body 110, such as shown in Figure 4A. Instead of extending in an axial direction parallel with the first axis of rotation, it is also to be appreciated that the strips 112 may extend in a circumferential direction around the first axis of rotation 104, such as shown in Figure 4B. In some configurations, the strips 112 extend in both an axial direction parallel with the first axis of rotation 104 and in a circumferential direction around the first axis of rotation 104, such as shown in Figure 4C. It is also to be appreciated that the strips 112 may be configured with substantially constant widths and in some configurations, the strips 112 may be configured with varying widths, such as shown in Figure 4D. In some configurations, such as shown in Figures 4E and 4F, the anvil may be configured to include anchor strips 112a that extend between and connect with the strips 112. The anchor strips 112a may help hold the strips 112 in position on the anvil 100.

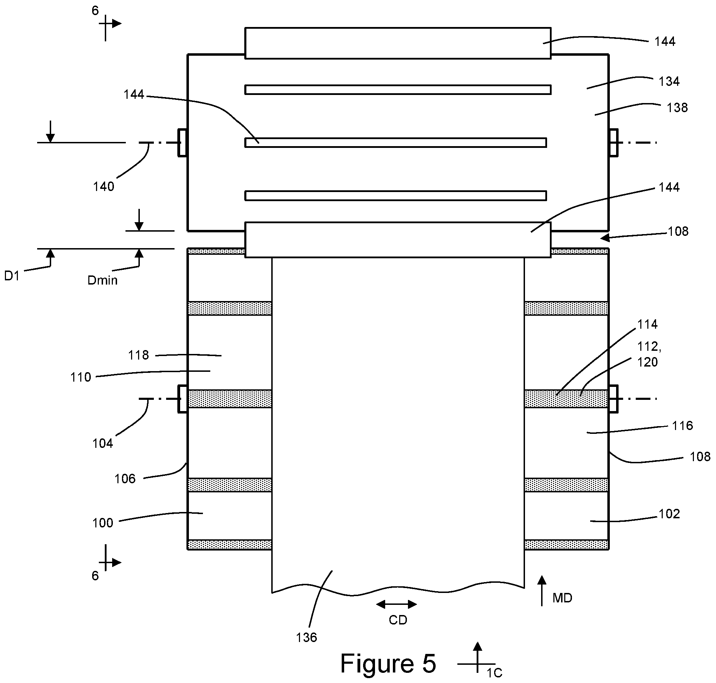

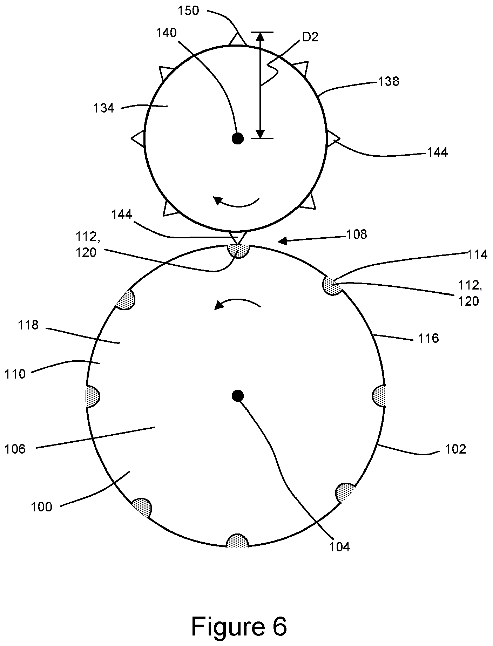

As previously mentioned, the anvil roll 100 may be used in combination with a tool member 134, such as shown in Figure 5, to perform various types of manufacturing operations on an advancing substrate 136. As shown in Figures 5 and 6, the tool member 134 may include an outer circumferential surface 138 and may be adapted to rotate about a second axis of rotation 140. The tool member 134 may be positioned adjacent the anvil roll 100 to define a nip 142 that may defined by a minimum distance, Dmin, between the outer circumferential surface 138 of the tool member 134 and the outer circumferential surface 102 of the anvil roll 100. The tool member 134 and the anvil roll 100 may be adapted to rotate in opposite directions such that tool member 134 contacts on the first portion 114 of the outer circumferential surface 102 of the anvil roll 100 defined by the strips 112 of abrasion resistant material 120. In some configurations, the tool member 134 may include one or more implements 144 that intermittently contact the strips 112 of abrasion resistant material 120 during rotation of the tool member 134 and anvil roll 100. As shown in Figure 5, the substrate 136 may advance in the machine direction MD through the nip 108 such that the substrate 136 is impinged upon between the implements 144 and the strips 112 of abrasion resistant material 120.

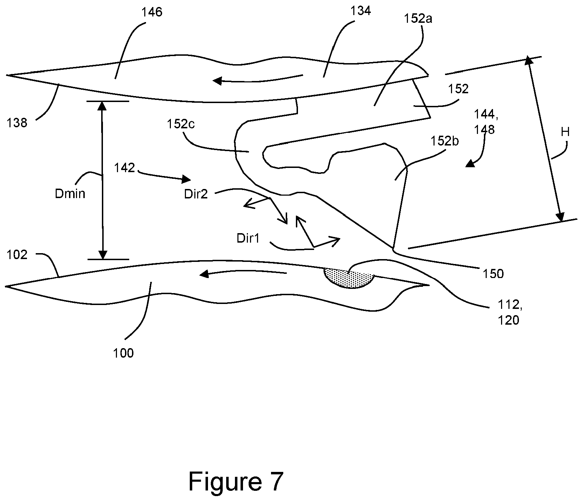

It is to be appreciated that the tool member 134 may be configured to perform various types of converting operations on the substrate 136, such as for example, cutting, embossing, and bonding, as one or more substrates 136 advance through the nip 108. Various examples of tool member configurations that may be used with the anvil rolls 100 herein are described in U.S. Patent Nos. 4,493,868; 4,854,984; 5,620,779; 5,798,167; 6,244,148; 6,248,195; 7,777,094; 7,861,756; and 8,440,043; and U.S. Patent Publication Nos. 2012/0079926 A1; 2013/0213547 A1; 2013/0218116 A1; 2014/0377513 A1; and 2014/0377506 A1; and European Patent Publication No. EP1635750B1. For example, Figure 7 shows the tool member 134 configured as a cutting roll 146 including one or more implements 144 comprising blades 148. As previously mentioned, the tool member 134 or cutting roll 146 may be adjacent to the anvil roll 100 and create a nip 142 defined by a minimum distance, Dmin, between the outer circumferential surface 138 of the tool member 134 or cutting roll 146 and the outer circumferential surface 102 of the anvil roll 100. In addition, the implements 144 or blades 148 may extend radially outward from the outer circumferential surface 138 of the cutting roll 134 to a distal edge 150 by a distance, H. And the distance, H, of each blade 148 is greater than the distance, Dmin, between the cutting roll 134 and the anvil roll 100, creating an interference distance between the distal edges 150 of the blades 150 and the outer circumferential surface 102 of the anvil roll 100. As such, in some embodiments the interference distance may be defined by the difference between the distance, H, and the distance, Dmin. As shown in Figures 5 and 6, it is to be appreciated that the interference distance between the implement 144 or blade 148 and the anvil roll 100 for the apparatuses and methods herein may also be defined with reference to a distance, D1, and a distance, D2, wherein D2 is greater than D1. With continued reference to Figures 5 and 6, the distance, D1, is the minimum distance between the axis of rotation 140 of the tool member 134 and the outer circumferential surface 102 of the anvil roll 100. And the distance, D2, is the maximum distance between the axis of rotation 140 of the tool member 134 and the distal edge 150 of the implement 144. As such, the interference distance may be defined by the difference between the distance, D2, and the distance, D1.

With continued reference to Figure 7, the blade 148 may also include a support member 152. The support member 152 may include a first, proximal, portion 152a; a second, distal, portion 152b; and a third, central flexible, portion 152c. As illustrated, the flexible portion 152c connects the distal portion 152b with the proximal portion 152a. The flexible portion 152c may flex and/or bend to allow the blade 148 to deflect when moving through the nip 142. As the blades 148 move though the nip 142 between the cutting roll 146 and the anvil roll 100, the interference distance may cause the flexible portion 152c of the support member to flex or bend, which in turn, allows the blade edges 150 to deflect in directions generally indicated by the arrows labeled as Dir1. As such, the bending and/or deformation of the flexible portion 152c causes the distal edges 150 of the blades 148 to exert pressure on the substrate 136 advancing through the nip 142 between distal edges 150 and the strips 112 of abrasion resistant material 120. As the cutting roll 146 continues to rotate and the blades 148 move away from the nip 142, the flexible portions 152c of the support members 152 return to the original uncompressed states before entering the nip 142, which may cause the distal edges 150 to move back in directions generally indicated by the arrows labeled as Dir2. It is to be appreciated that various embodiments of blade assembly configurations having flexible blades and/or flexible support members and/or springs have been disclosed herein. Such blade assembly configurations may provide relatively more simple machine design and fabrication requirements than those that may be required for other designs, such as rigid die cutter designs. For example, the presently disclosed blade assembly configurations may provide for greater flexibility in knife set-up and may reduce the level of precision that would otherwise be required for rigid die cutter configurations.

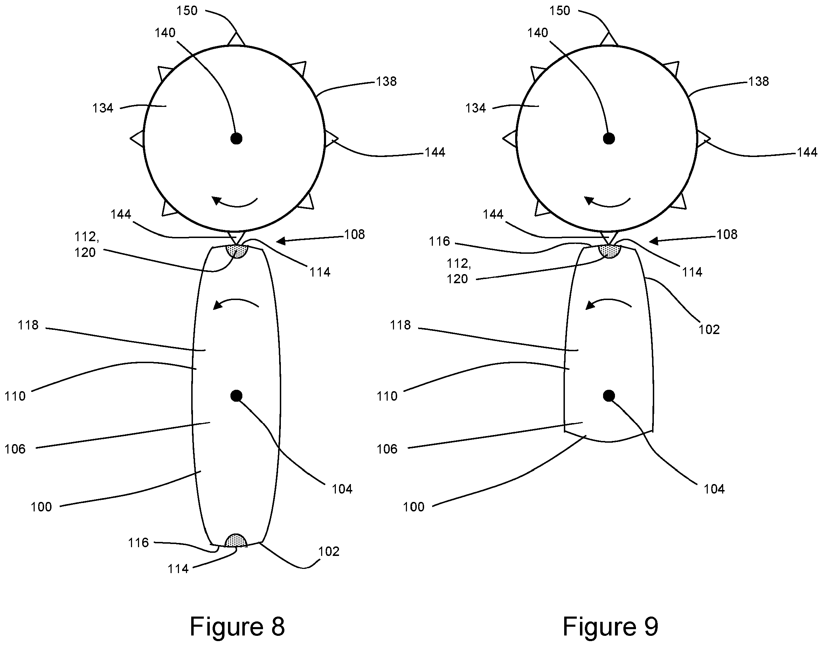

It is to be appreciated that the anvil roll and components such as the body and/or grooves may be configured in various ways. In some configurations, the body and anvil may define shapes than a generally cylindrical shape. For example, Figure 8 shows an anvil roll 100 including a body 110 that is elongate and may include strips 112 of abrasion resistant material 120 fused to opposing end portions of the body 110. In another example shown in Figure 9, the anvil roll 100 includes a body 110 that is elongate and includes a single strip 112 of abrasion resistant material 120 fused to an end portion of the body 110.

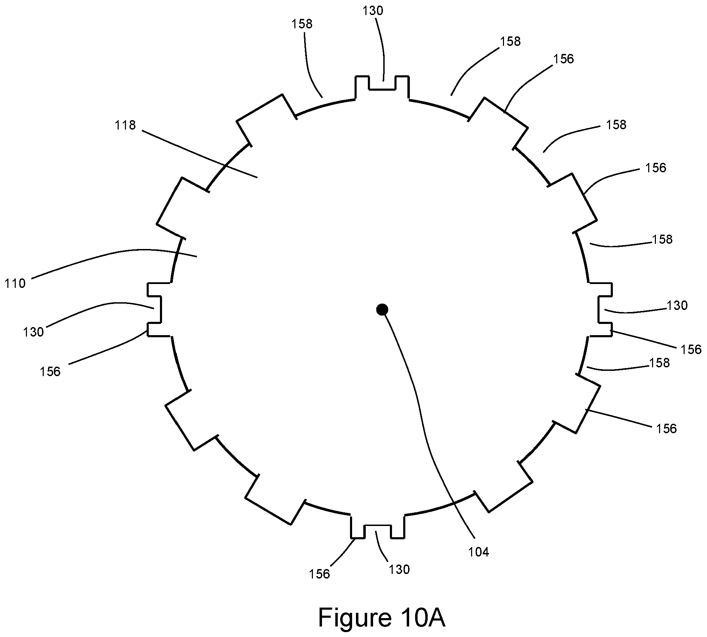

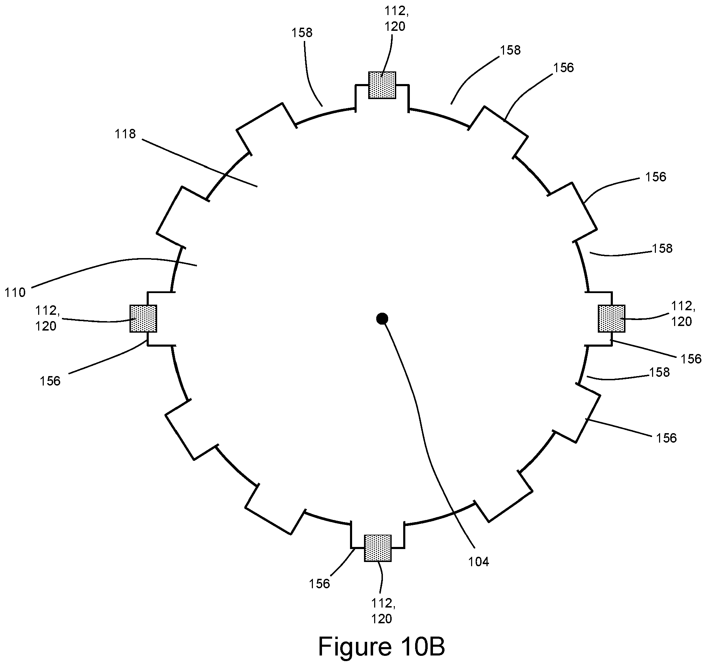

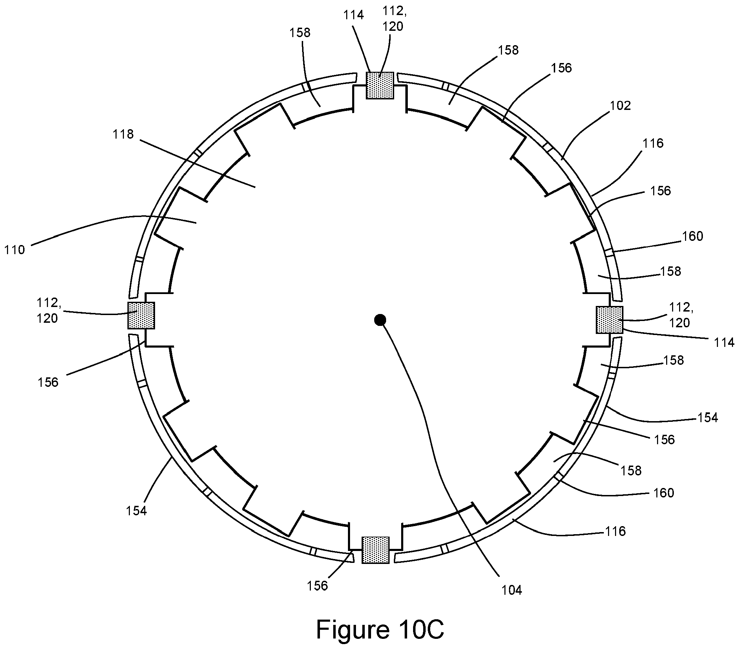

In another example such as shown in Figures 10A-10C, the anvil roll 100 may also be configured with one or more shell members 154 connected with the body 110. With reference to Figure 10A, the body 110 may include one or more radially projecting platforms 156 that are circumferentially spaced from one another such that channels 158 are defined between adjacent platforms 156. And one or more grooves 130 may also be defined in one or more platforms 156. As shown in Figure 10B and as discussed above, one or more abrasion resistant materials 120 are fused to the body 110 in the grooves 130 to form strips 112. Thus, as discussed above, the body 110 may be from a first material 118 and the strips 112 are made from one or more abrasion resistant materials 120 that are different from the first material 118. The strips 120 may also be configured to project radially outward from the platforms 156. As shown in Figure 10C, one or more shell members 154 may be connected with the body. For example, shell members 154 may positioned on opposing sides of the strips 112. Thus, the strips 112 may define a first portion 114 of the outer circumferential surface 102 of the anvil roll 100 and the shell members 154 define a second portion 116 of the outer circumferential surface 102. In addition, the shell members 154 may include apertures 160. In turn, the apertures 160 may be positioned such that when the shell members 154 are connected with the body 110, the apertures 160 may be in fluid communication with the channels 158. As such, the anvil roll 100 may be connected with a vacuum pressure source that is in fluid communication with the channels 158 and apertures 160. It is to be appreciated that the shell members 154 may be connected with the body 110 in various ways. For example, the shell members 154 may be bolted to the body 110 to allow for ease of assembly and removal for replacement and/or maintenance purposes.

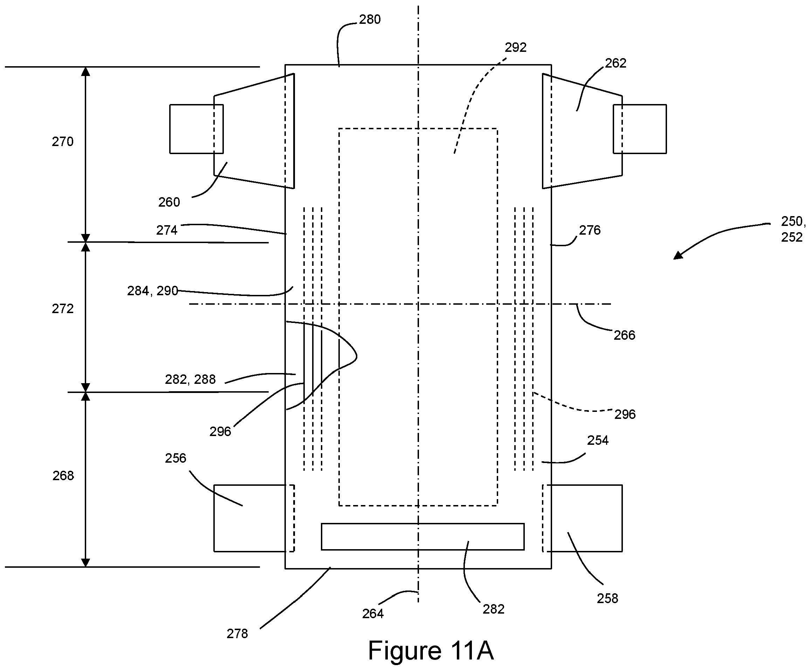

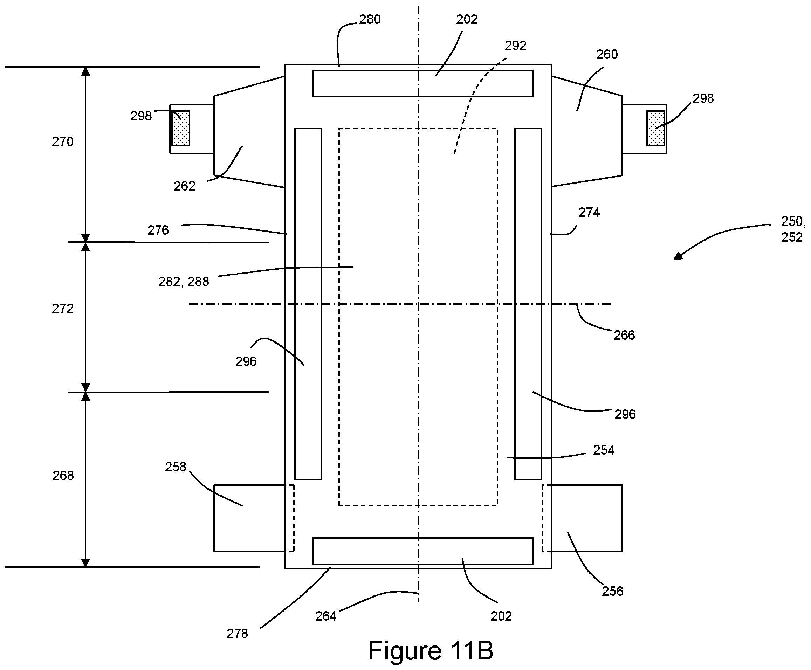

As mentioned above, apparatuses and methods of the present disclosure may be utilized to perform various manufacturing operations on substrates used in the manufacture of absorbent articles. Such substrates may be utilized in absorbent article components such as, for example: backsheets, topsheets, absorbent cores, front and/or back ears, fastener components, and various types of elastic webs and components such as leg elastics, barrier leg cuff elastics, and waist elastics. For the purposes of a specific illustration, Figures 11A and 11B show an example of a disposable absorbent article 250 in the form of a diaper 252 that may be constructed from such substrates and components manipulated during manufacture according to the apparatuses and methods disclosed herein. In particular, Figure 11A is a partially cut away plan view of an absorbent article in the form of a taped diaper that may include one or more substrates and components manipulated during manufacture according to the apparatuses and methods disclosed herein with the portion of the diaper that faces away from a wearer oriented towards the viewer. Figure 11B is a plan view of the absorbent article of Figure 11A that may include one or more substrates and components manipulated during manufacture according to the apparatuses and methods disclosed herein with the portion of the diaper that faces toward a wearer oriented towards the viewer.

As shown in Figures 11A-11B, the diaper 252 includes a chassis 254 having a first ear 256, a second ear 258, a third ear 260, and a fourth ear 262. To provide a frame of reference for the present discussion, the chassis is shown with a longitudinal axis 264 and a lateral axis 266. The chassis 254 is shown as having a first waist region 268, a second waist region 270, and a crotch region 272 disposed intermediate the first and second waist regions. The periphery of the diaper is defined by a pair of longitudinally extending side edges 274, 276; a first outer edge 278 extending laterally adjacent the first waist region 268; and a second outer edge 280 extending laterally adjacent the second waist region 270. As shown in Figures 11A-11B, the chassis 254 includes an inner, body-facing surface 282, and an outer, garment-facing surface 284. A portion of the chassis structure is cut-away in Figures 11A-11B to more clearly show the construction of and various features that may be included in the diaper. As shown in Figures 11A-11B, the chassis 254 of the diaper 252 may include a topsheet 288 defining the inner, body-facing surface 282, and a backsheet 290 defining the outer, garment-facing surface 284. An absorbent core 292 may be disposed between a portion of the topsheet 288 and the backsheet 290. As discussed in more detail below, any one or more of the regions may be stretchable and may include an elastomeric material or laminate as described herein. As such, the diaper 252 may be configured to adapt to a specific wearer's anatomy upon application and to maintain coordination with the wearer's anatomy during wear.

The absorbent article 250 may also include an elastic waist feature 202 shown in Figures 11A-11B in the form of a waist band and may provide improved fit and waste containment. The elastic waist feature 202 may be configured to elastically expand and contract to dynamically fit the wearer's waist. The elastic waist feature 202 can be incorporated into the diaper and may extend at least longitudinally outwardly from the absorbent core 292 and generally form at least a portion of the first and/or second outer edges 278, 280 of the diaper 252. In addition, the elastic waist feature may extend laterally to include the ears. While the elastic waist feature 202 or any constituent elements thereof may comprise one or more separate elements affixed to the diaper, the elastic waist feature may be constructed as an extension of other elements of the diaper, such as the backsheet 290, the topsheet 288, or both the backsheet and the topsheet. In addition, the elastic waist feature 202 may be disposed on the outer, garment-facing surface 284 of the chassis 254; the inner, body-facing surface 282; or between the inner and outer facing surfaces. The elastic waist feature 202 may be constructed in a number of different configurations including those described in U.S. Patent Publication Nos. 2007/0142806 A1; 2007/0142798 A1; and 2007/0287983 A1.

As shown in Figures 11A-11B, the diaper 252 may include leg cuffs 296 that may provide improved containment of liquids and other body exudates. In particular, elastic gasketing leg cuffs can provide a sealing effect around the wearer's thighs to prevent leakage. It is to be appreciated that when the diaper is worn, the leg cuffs maybe placed in contact with the wearer's thighs, and the extent of that contact and contact pressure may be determined in part by the orientation of diaper on the body of the wearer. The leg cuffs 296 may be disposed in various ways on the diaper 202.

The diaper 252 may be provided in the form of a pant-type diaper or may alternatively be provided with a re-closable fastening system, which may include fastener elements in various locations to help secure the diaper in position on the wearer. For example, fastener elements 298 may be located on the ears and may be adapted to releasably connect with one or more corresponding fastening elements located in the first or second waist regions. For example, as shown in Figure 11A, the diaper 252 may include a connection zone 282, sometimes referred to as a landing zone, in the first waist region 268. It is to be appreciated that various types of fastening elements may be used with the diaper.

It is to be appreciated that the apparatuses and methods herein may be used to provide for the cutting and removal of trim material from advancing substrates and components during the manufacture of absorbent articles, such as the diaper of Figures 11A-11B. For example, the trim removal apparatus may be used to remove trim material during the manufacture of a topsheet, a backsheet, an absorbent core, an ear, and fastening elements.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

While particular embodiments of the present invention have been illustrated and described, it would be obvious to those skilled in the art that various other changes and modifications can be made without departing from the scope of the invention as defined by the claims.

## Claims

1. An apparatus comprising:
an anvil roll (100) comprising an outer circumferential surface (102) and adapted to rotate about a first axis of rotation (104), and
a tool member (134) adjacent the anvil roll (100) and adapted to rotate about a second axis of rotation (140) and to contact the first portion (114) of the outer circumferential surface (102) of the anvil roll (100);
the anvil roll (100) comprising:
a body (110) consisting of a first material (118), wherein the first material (118) is selected from the group consisting of: an iron alloy, an aluminum alloy, and a titanium alloy, and
wherein the body comprises:
a groove (130); and
**characterized by**
one or more abrasion resistant materials (120) fused to the body (110) with a laser cladding process and filling the groove (130) to form a strip (112), the one or more abrasion resistant materials (120) being different from the first material (118), wherein, during the laser cladding process, the first material (118) of the body (110) is partially melted during deposition of the abrasion resistant material (120) into the grooves (130) to create a metallurgical bond between the abrasion resistant material (120) and the first material (118) of the body (110);
wherein a first portion (114) of the outer circumferential surface (102) of the anvil roll (100) is defined by the strip (112).

2. The apparatus according to claim 1, wherein the body comprises a channel (158).

3. The apparatus according to claim 2, further comprising a shell member (154) connected with body (110), wherein a second portion (116) of the outer circumferential surface (102) of the anvil roll (100) is defined by the shell member (154) and comprising holes (160) in the shell member (154); and a vacuum pressure source in fluid communication with the holes (160) and the channel (158).

4. The apparatus according to any one of claims 1-3, wherein a second portion (116) of the outer circumferential surface (102) is defined by the body (110).

5. The apparatus according to any one of claims 1-5, further comprising:
a first hole (122) comprising a first perimeter (124) in the outer circumferential surface (102) and extending radially inward from the outer circumferential surface (102);
a second hole (122) comprising a second perimeter (124) in the outer circumferential surface (102) and extending radially inward from the outer circumferential surface (102); and
wherein the groove (130) separates the first perimeter from the second perimeter.

6. The apparatus according to claim 5, further comprising a vacuum pressure source in fluid communication with at least one of the first hole (122) and the second hole (122).

7. The apparatus according to any one of claims 1-6, wherein the one or more abrasion resistant materials (120) comprises at least one of: powder-metallurgical steel; titanium carbide, niobium carbide, tantalum carbide, chromium carbide, tungsten carbide, and a carbide of at least one element of the fourth, the fifth, the sixth and/or the seventh group of the periodic table.

8. The apparatus according to any one of claims 1-7, wherein the iron alloy is selected from the group consisting of: stainless steel and tool steel.

9. The apparatus according to any one of claims 1-8, wherein the strip (112) comprises a radial depth of greater than about 2 mm and less than about 4 mm.

10. The apparatus according to any one of claims 1-9, wherein the strip (112) extends for a length in a circumferential direction around the first axis of rotation.

11. The apparatus according to any one of claims 1-10, wherein the body (110) defines a length L extending axially along the first axis of rotation (104), and wherein the strip (112) extends axially for the length L.

12. The apparatus according to any one of claims 1-11, wherein the tool member (134) comprises a (134) cutting roll (146) adjacent the anvil roll (100) to define a nip (142) between the anvil roll (100) and the cutting roll (146), wherein the outer circumferential surface (102) of the anvil roll (100) and the second axis of rotation (140) are separated by a minimum distance D1;
the cutting roll (146) comprising a blade (148) comprising a distal edge (150), and wherein the distal edge (150) and the second axis of rotation (140) are separated by a distance D2, wherein D2 is greater than D1 and defining an interference distance equal to the difference between D2 and D1; and
wherein the distal edge (150) of the blade (148) is adapted to deflect the interference distance when contacting the first portion (114) of the outer circumferential surface (102) of the anvil roll (100) while moving through the nip (142).

13. The apparatus of claim 12, wherein the blade (150) comprises a support member (152), wherein the support member (152) bends when the distal edge (150) deflects by the interference distance.

14. The apparatus of claim 12, wherein the blade (148) bends when contacting the first portion (114) of the outer circumferential surface (102) of the anvil roll (100).

15. The apparatus according to any one of claims 1-14, wherein the tool member (134) comprises a bonding roll, the bonding roll comprising a pattern element that contacts the first portion (114) of the outer circumferential surface (102) of the anvil roll (100).

## Patentansprüche

1. Apparat, umfassend:
eine Ambosswalze (100), die eine äußere Umfangsoberfläche (102) umfasst und dazu konzipiert ist, sich um eine erste Drehachse (104) zu drehen, und
ein Werkzeugelement (134), das zu der Ambosswalze (100) benachbart und dazu konzipiert ist, sich um eine zweite Drehachse (140) zu drehen und den ersten Abschnitt (114) der äußeren Umfangsoberfläche (102) der Ambosswalze (100) zu berühren;
wobei die Ambosswalze (100) umfasst:
einen Körper (110), der aus einem ersten Material (118) besteht, wobei das erste Material (118) ausgewählt ist aus der Gruppe bestehend aus: einer Eisenlegierung, einer Aluminiumlegierung und einer Titanlegierung, und wobei der Körper umfasst:
eine Rille (130); und
**gekennzeichnet durch**
ein oder mehrere abnutzungsbeständige Materialien (120), die durch einen Laserplattierprozess mit dem Körper (110) verschmolzen sind und die Rille (130) füllen, um einen Streifen (112) zu bilden, wobei sich das eine oder die mehreren abnutzungsbeständigen Materialien (120) von dem ersten Material (118) unterscheiden, wobei während des Laserplattierprozesses das erste Material (118) des Körpers (110) während der Abscheidung des abnutzungsbeständigen Materials (120) in die Rillen (130) teilweise aufgeschmolzen wird, um eine metallurgische Bindung zwischen dem abnutzungsbeständigen Material (120) und dem ersten Material (118) des Körpers (110) zu bilden;
wobei ein erster Abschnitt (114) der äußeren Umfangsoberfläche (102) der Ambosswalze (100) durch den Streifen (112) bestimmt ist.

2. Apparat nach Anspruch 1, wobei der Körper einen Kanal (158) umfasst.

3. Apparat nach Anspruch 2, ferner umfassend ein Schalenelement (154), das mit dem Körper (110) verbunden ist, wobei ein zweiter Abschnitt (116) der äußeren Umfangsoberfläche (102) der Ambosswalze (100) durch das Schalenelement (154) bestimmt ist und Löcher (160) in dem Schalenelement (154) umfasst; und eine Vakuumdruckquelle in Fluidaustausch mit den Löchern (160) und dem Kanal (158).

4. Apparat nach einem der Ansprüche 1-3, wobei ein zweiter Abschnitt (116) der äußeren Umfangsoberfläche (102) durch den Körper (110) bestimmt ist.

5. Apparat nach einem der Ansprüche 1-5, ferner umfassend:
ein erstes Loch (122), das einen ersten Umfang (124) in der äußeren Umfangsoberfläche (102) umfasst und sich von der äußeren Umfangsoberfläche (102) radial nach innen erstreckt;
ein zweites Loch (122), das einen zweiten Umfang (124) in der äußeren Umfangsoberfläche (102) umfasst und sich von der äußeren Umfangsoberfläche (102) radial nach innen erstreckt; und
wobei die Rille (130) den ersten Umfang von dem zweiten Umfang trennt.

6. Apparat nach Anspruch 5, ferner umfassend eine Vakuumdruckquelle in Fluidaustausch mit wenigstens einem von dem ersten Loch (122) und dem zweiten Loch (122).

7. Apparat nach einem der Ansprüche 1-6, wobei das eine oder die mehreren abnutzungsbeständigen Materialien (120) wenigstens eines umfassen von: pulvermetallurgischem Stahl; Titancarbid, Niobcarbid, Tantalcarbid, Chromcarbid, Wolframcarbid und einem Carbid aus wenigstens einem Element der vierten, der fünften, der sechsten und/oder der siebten Gruppe des Periodensystems.

8. Apparat nach einem der Ansprüche 1-7, wobei die Eisenlegierung ausgewählt ist aus der Gruppe bestehend aus: Edelstahl und Werkzeugstahl.

9. Apparat nach einem der Ansprüche 1-8, wobei der Streifen (112) eine radiale Tiefe von mehr als etwa 2 mm und weniger als etwa 4 mm umfasst.

10. Apparat nach einem der Ansprüche 1-9, wobei sich der Streifen (112) über eine Länge in einer Umfangsrichtung um die erste Drehachse erstreckt.

11. Apparat nach einem der Ansprüche 1-10, wobei der Körper (110) eine Länge L bestimmt, die sich axial entlang der ersten Drehachse (104) erstreckt, und wobei sich der Streifen (112) axial über die Länge L erstreckt.

12. Apparat nach einem der Ansprüche 1-11, wobei das Werkzeugelement (134) eine (134) Schneidwalze (146) benachbart zu der Ambosswalze (100) umfasst, um einen Walzenspalt (142) zwischen der Ambosswalze (100) und der Schneidwalze (146) zu bestimmen, wobei die äußere Umfangsoberfläche (102) der Ambosswalze (100) und die zweite Drehachse (140) durch einen minimalen Abstand D1 getrennt sind;
wobei die Schneidwalze (146) eine Klinge (148) umfasst, die eine distale Kante (150) umfasst, und wobei die distale Kante (150) und die zweite Drehachse (140) durch einen Abstand D2 getrennt sind, wobei D2 größer als D1 ist und einen Interferenzabstand bestimmt, der gleich der Differenz zwischen D2 und D1 ist; und
wobei die distale Kante (150) der Klinge (148) dazu konzipiert ist, beim Kontaktieren des ersten Abschnitts (114) der äußeren Umfangsoberfläche (102) der Ambosswalze (100) um den Interferenzabstand auszulenken, während sie sich durch den Walzenspalt (142) bewegt.

13. Apparat nach Anspruch 12, wobei die Klinge (150) ein Stützelement (152) umfasst, wobei sich das Stützelement (152) biegt, wenn die distale Kante (150) um den Interferenzabstand ausgelenkt wird.

14. Apparat nach Anspruch 12, wobei sich die Klinge (148) beim Kontaktieren des ersten Abschnitts (114) der äußeren Umfangsoberfläche (102) der Ambosswalze (100) biegt.

15. Apparat nach einem der Ansprüche 1-14, wobei das Werkzeugelement (134) eine Bonding-Walze umfasst, wobei die Bonding-Walze ein Musterelement umfasst, das den ersten Abschnitt (114) der äußeren Umfangsoberfläche (102) der Ambosswalze (100) berührt.

## Revendications

1. Appareil comprenant :
un rouleau enclume (100) comprenant une surface circonférentielle externe (102) et conçu pour se mettre en rotation autour d'un premier axe de rotation (104), et
un élément d'outil (134) adjacent au rouleau enclume (100) et conçu pour se mettre en rotation autour d'un deuxième axe de rotation (140) et pour venir en contact avec la première partie (114) de la surface circonférentielle externe (102) du rouleau enclume (100) ;
le rouleau enclume (100) comprenant :
un corps (110) constitué d'un premier matériau (118), dans lequel le premier matériau (118) est choisi dans le groupe constitué : d'un alliage de fer, d'un alliage d'aluminium, et d'un alliage de titane, et dans lequel le corps comprend :
une rainure (130) ; et
**caractérisé par**
un ou plusieurs matériaux résistant à l'abrasion (120) fusionnés au corps (110) avec un processus de revêtement au laser et remplissant la rainure (130) pour former une bande (112), le ou les matériaux résistant à l'abrasion (120) étant différents du premier matériau (118), dans lequel, pendant le processus de revêtement au laser, le premier matériau (118) du corps (110) est partiellement fondu pendant le dépôt du matériau résistant à l'abrasion (120) dans les rainures (130) pour créer une liaison métallurgique entre le matériau résistant à l'abrasion (120) et le premier matériau (118) du corps (110) ;
dans lequel une première partie (114) de la surface circonférentielle externe (102) du rouleau enclume (100) est définie par la bande (112).

2. Appareil selon la revendication 1, dans lequel le corps comprend un canal (158).

3. Appareil selon la revendication 2, comprenant en outre un élément d'enveloppe (154) connecté au corps (110), dans lequel une deuxième partie (116) de la surface circonférentielle externe (102) du rouleau enclume (100) est définie par l'élément d'enveloppe (154) et comprenant des trous (160) dans l'élément d'enveloppe (154) ; et une source de dépression en communication fluidique avec les trous (160) et le canal (158).

4. Appareil selon l'une quelconque des revendications 1 à 3, dans lequel une deuxième partie (116) de la surface circonférentielle externe (102) est définie par le corps (110).

5. Appareil selon l'une quelconque des revendications 1 à 5, comprenant en outre :
un premier trou (122) comprenant un premier périmètre (124) dans la surface circonférentielle externe (102) et s'étendant radialement vers l'intérieur à partir de la surface circonférentielle externe (102) ;
un deuxième trou (122) comprenant un deuxième périmètre (124) dans la surface circonférentielle externe (102) et s'étendant radialement vers l'intérieur à partir de la surface circonférentielle externe (102) ; et
dans lequel la rainure (130) sépare le premier périmètre du deuxième périmètre.

6. Appareil selon la revendication 5, comprenant en outre une source de dépression en communication fluidique avec au moins un du premier trou (122) et du deuxième trou (122).

7. Appareil selon l'une quelconque des revendications 1 à 6, dans lequel le ou les matériaux résistant à l'abrasion (120) comprennent au moins un parmi : acier métallurgique en poudre ; carbure de titane, carbure de niobium, carbure de tantale, carbure de chrome, carbure de tungstène, et un carbure d'au moins un élément du quatrième, du cinquième, du sixième et/ou du septième groupe du tableau périodique.

8. Appareil selon l'une quelconque des revendications 1 à 7, dans lequel l'alliage de fer est choisi dans le groupe constitué de : acier inoxydable et acier à outil.

9. Appareil selon l'une quelconque des revendications 1 à 8, dans lequel la bande (112) comprend une profondeur radiale supérieure à environ 2 mm et inférieure à environ 4 mm.

10. Appareil selon l'une quelconque des revendications 1 à 9, dans lequel la bande (112) s'étend sur une longueur dans une direction circonférentielle autour du premier axe de rotation.

11. Appareil selon l'une quelconque des revendications 1 à 10, dans lequel le corps (110) définit une longueur L s'étendant axialement le long du premier axe de rotation (104), et dans lequel la bande (112) s'étend axialement sur la longueur L.

12. Appareil selon l'une quelconque des revendications 1 à 11, dans lequel l'élément d'outil (134) comprend un (134) rouleau de coupe (146) adjacent au rouleau enclume (100) pour définir une ligne de contact (142) entre le rouleau enclume (100) et le rouleau de coupe (146), dans lequel la surface circonférentielle externe (102) du rouleau enclume (100) et le deuxième axe de rotation (140) sont séparés par une distance minimale D1 ;
le rouleau de coupe (146) comprenant une lame (148) comprenant un bord distal (150), et dans lequel le bord distal (150) et le deuxième axe de rotation (140) sont séparés par une distance D2, dans lequel D2 est supérieur à D1 et définissent une distance d'interférence égale à la différence entre D2 et D1 ; et
dans lequel le bord distal (150) de la lame (148) est conçu pour dévier la distance d'interférence lors d'un contact avec la première partie (114) de la surface circonférentielle externe (102) du rouleau enclume (100) en se déplaçant à travers la ligne de contact (142).

13. Appareil selon la revendication 12, dans lequel la lame (150) comprend un élément de support (152), dans lequel l'élément de support (152) fléchit lorsque le bord distal (150) dévie de la distance d'interférence.

14. Appareil selon la revendication 12, dans lequel la lame (148) fléchit lorsqu'elle vient en contact avec la première partie (114) de la surface circonférentielle externe (102) du rouleau enclume (100).

15. Appareil selon l'une quelconque des revendications 1 à 14, dans lequel l'élément d'outil (134) comprend un rouleau de liaison, le rouleau de liaison comprenant un élément de motif qui vient en contact avec la première partie (114) de la surface circonférentielle externe (102) du rouleau enclume (100).
